# EUROPEAN PATENT SPECIFICATION

(11) **EP 4 026 527 B1**
(45) Date of publication and mention of the grant of the patent: **02.04.2025**
(21) Application number: 20893370.5
(22) Date of filing: 20.11.2020
(51) Int. Cl.: A61F 13/496, A61F 13/511, A61F 13/514

(54) **UNDERPANTS-TYPE DISPOSABLE DIAPER**
UNTERHOSENARTIGE WEGWERFWINDEL
COUCHE JETABLE DE TYPE CULOTTE

(30) Priority: 29.11.2019 JP 2019217269
(43) Date of publication of application: 13.07.2022
(73) Proprietor: Unicharm Corporation, Ehime 7990111 (JP)
(72) Inventor: ICHIKAWA, Makoto, Kanonji-shi, Kagawa 769-1602 (JP); MUKAI, Hirotomo, Kanonji-shi, Kagawa 769-1602 (JP); TANAKA, Suguru, Kanonji-shi, Kagawa 769-1602 (JP); OKUNO, Shingo, Kanonji-shi, Kagawa 769-1602 (JP)
(74) Representative: Dolleymores
(86) International application number: PCT/JP2020/043441
(87) International publication number: WO 2021/106794

(56) References cited:
- EP-A2- 0 187 728
- WO-A1-96/11656
- JP-A- 2001 314 441
- JP-A- 2014 195 747
- JP-A- 2017 029 683
- JP-A- 2017 051 485
- JP-A- 2020 000 718
- JP-A- H08 182 704
- US-A1- 2017 246 047

## Description

### FIELD

The present invention relates to an underpants-shaped disposable diaper.

### BACKGROUND

An underpants-shaped disposable diaper is known as an absorbent article. For example, Patent Literature 1 discloses an underpants-shaped disposable diaper in which a waist opening is formed by joining front and back belt portions (6, 7) at two side portions by side-edge joining portions (10). In such an underpants-shaped disposable diaper, when removing the soiled diaper after use from the wearer's body, it is common to open the waist opening and remove the diaper by peeling locking portions (side-edge joining portions) on two side portions of the waist portion (belt portion). In addition, when peeling off the locking portion, a front waist portion and a back waist portion are pulled in opposite directions (for example, in a front-back direction) along the locking portion, thereby peeling off the locking.

Other prior art arrangements of underpants-shaped disposable diapers are known from WO 96/11656 A1, EP 0 187 728 A2 and US 2017/246047 A1.

### [CITATION LIST]

### [PATENT LITERATURE]

Patent Literature 1: Japanese Patent Application Publication No.2009-61051

### SUMMARY

### [TECHNICAL PROBLEM]

In recent years, from viewpoints of reducing in material cost, comfort to wear, and breathability, there has been an increasing need for a thinner material. There is a demand to use a sheet member (for example, nonwoven fabric) whose basis weight is as low as possible for a material constituting waist portions of a diaper. However, decreasing the basis weight of the sheet member also decreases a material strength of the sheet member, and accordingly, in some cases, a part of the sheet member is torn along a lateral direction at a time during peeling off the locking portion. In this case, there is a risk that the diaper cannot be removed or that excrement adhering to the inside of the diaper leaks outside.

The present invention was achieved in light of conventional problems such as that described above and an aspect of the present invention is, in an underpants-shaped disposable diaper formed of a material having a low basis weight, to suppress laterally tearing of a material that constitutes a front waist portion and a back waist portion, when peeling off a locking portion of the waist portions.

### [SOLUTION TO PROBLEM]

A main aspect of the present invention for achieving the above-described aspect is a underpants-shaped disposable diaper having a vertical direction, a lateral direction, and a front-back direction that intersect each other, the underpants-shaped disposable diaper including: a liquid-absorbent absorbent main body; a front waist portion and a back waist portion that are provided on a non-skin side of the absorbent main body; and a pair of locking portions that lock two lateral end portions of the front waist portion and two lateral end portions of the back waist portion, in each of the front waist portion and the back waist portion, a sum of a basis weight of a skin-side sheet member arranged the farthest on a skin side and a basis weight of a sheet member arranged the farthest on a non-skin side being 32 g/m² or less, the skin-side sheet member overlapping the locking portions in two end portions in the lateral direction, the skin-side sheet member including a non-joining portion in a region adjacent to inside of the locking portion in the lateral direction, the non-joining portion being a portion that is not joined to a sheet member overlaid on a non-skin side of the skin-side sheet member, a tearing strength for tearing the skin-side sheet member along the lateral direction being greater than a peeling strength of the locking portion.

Features of the present invention other than the above will become clear by reading the description of the present specification with reference to the accompanying drawings.

### [ADVANTAGEOUS EFFECT OF THE INVENTION]

According to the present invention, in the underpants-shaped disposable diaper using a material having a low basis weight, it is possible to suppress the tearing of the material that constitutes the front waist portion and the back waist portion, in the lateral direction, when peeling off the locking portion of the waist portions.

### BRIEF DESCRIPTION OF DRAWINGS

FIG. 1 is a schematic front view of a diaper 1.
FIG. 2 is a schematic plan view of the diaper 1 in an unfolded and stretched state.
FIG. 3 is a schematic cross-sectional view taken along a line I-I in FIG. 2.
FIG. 4 is a schematic cross-sectional view taken along a line A-A in FIG. 2.
FIG. 5 is a schematic view showing the diaper 1 in an underpants-shaped state when seen from one side (right side) in the lateral direction.
FIG. 6A is a schematic diagram illustrating peeling of a locking portion 60.
FIG. 6B is a schematic diagram illustrating the peeling of the locking portion 60.
FIG. 7 is a schematic cross-sectional view taken along a line C-C in FIG. 5.
FIG. 8 is a schematic enlarged view of a region D (locking portion 60) in FIG. 7.
FIG. 9 is a diagram illustrating a test piece used for measuring the tearing strength of the skin-side sheet member.
FIG. 10 is a conceptual diagram illustrating a method for measuring the tearing strength of the skin-side sheet member.
FIG. 11 is a diagram illustrating a test piece used for measuring the peeling strength of the locking portion 60.
FIG. 12 is a table showing results of the measurement.
FIG. 13 is a schematic cross-sectional view showing how components of a front waist portion 20 are overlaid in a thickness direction.
FIG. 14A is an enlarged plan view illustrating the shape and arrangement of the locking portion 60.
FIG. 14B is an enlarged plan view illustrating the shape and arrangement of the locking portion 60.
FIG. 14C is an enlarged plan view illustrating the shape and arrangement of the locking portion 60.
FIG. 15A is a schematic cross-sectional view showing a modified example of the waist portion 20 (30).
FIG. 15B is a schematic cross-sectional view showing a modified example of the waist portion 20 (30).

### DESCRIPTION OF EMBODIMENTS

At least following matters will become clear with description of this specification and attached drawings.

A underpants-shaped disposable diaper having a vertical direction, a lateral direction, and a front-back direction that intersect each other, the underpants-shaped disposable diaper including: a liquid-absorbent absorbent main body; a front waist portion and a back waist portion that are provided on a non-skin side of the absorbent main body; and a pair of locking portions that lock two lateral end portions of the front waist portion and two lateral end portions of the back waist portion, in each of the front waist portion and the back waist portion, a sum of a basis weight of a skin-side sheet member arranged the farthest on a skin side and a basis weight of a sheet member arranged the farthest on a non-skin side being 32 g/m² or less, the skin-side sheet member overlapping the locking portions in two end portions in the lateral direction, the skin-side sheet member including a non-joining portion in a region adjacent to inside of the locking portion in the lateral direction, the non-joining portion being a portion that is not joined to a sheet member overlaid on a non-skin side of the skin-side sheet member, a tearing strength for tearing the skin-side sheet member along the lateral direction being greater than a peeling strength of the locking portion.

According to the underpants-shaped disposable diaper, the total of the basis weights of the skin-side sheet member and of the non-skin-side sheet member which constitute the waist portion is 32 g/m² or less, and athis makes it possible to realize a diaper having excellent manufacturing cost and breathability. In addition, even in the case of using a material having a low basis weight as described above, in an operation of peeling off the locking portion in order to remove the diaper from the wearer's body, it is possible to suppress the skin-side sheet member provided in the waist portion from being torn along the lateral direction.

In such a underpants-shaped disposable diaper, it is desirable that in at least one of an upper end region and a lower end region of the locking portion, of at least one of the front waist portion and the back waist portion, a basis weight of a sheet member arranged the farthest on the skin side is equal to or more than a basis weight of a sheet member arranged the farthest on the non-skin side.

According to the underpants-shaped disposable diaper, compared to the skin-side sheet member, a large force is less likely to act on the non-skin-side sheet member when peeling off the locking portion. For such a non-skin-side sheet member, it is possible to use a sheet member having a smaller basis weight than that of the skin-side sheet member. This makes it possible to realize a diaper in which the waist portions are less likely to be torn when peeling off the locking portions and whose manufacturing cost is low.

In such a underpants-shaped disposable diaper, it is desirable that in both the upper end region and the lower end region of the locking portion, of at least one of the front waist portion and the back waist portion, a basis weight of a sheet member arranged the farthest on the skin side is equal to or more than a basis weight of a sheet member arranged the farthest on the non-skin side.

According to the underpants-shaped disposable diaper, it possible to realize a diaper whose manufacturing cost is low and in which the waist portions are less likely to be torn in both cases of peeling off the locking portion from the upper side and peeling off the locking portion from the lower side, when removing the diaper from the wearer's body.

In such a underpants-shaped disposable diaper, it is desirable that the front waist portion and the back waist portion each have a waist elastic member, and that the waist elastic member is arranged on the non-skin side with respect to a central position in a thickness direction of at least one of the front waist portion and the back waist portion.

According to the underpants-shaped disposable diaper, in the waist portion, the distance between the waist elastic member and the wearer's skin increases, and accordingly, the waist elastic member can be suppressed from tightly digging into the wearer's skin due to a contractive force generated by the waist elastic member.

In such a underpants-shaped disposable diaper, it is desirable that in at least one of the front waist portion and the back waist portion, a thickness of a sheet member arranged the farthest on the skin side is equal to or more than a thickness of a sheet member arranged the farthest on the non-skin side.

According to the underpants-shaped disposable diaper, increase in thickness of the skin-side sheet member can further make it less likely to tear the skin-side sheet member at the time of peeling off the locking portion, while suppressing the tightening by the waist elastic member.

In such a underpants-shaped disposable diaper, it is desirable that an average value of a width of the locking portion in the lateral direction is 3 mm or less.

According to the underpants-shaped disposable diaper, the peeling strength of the locking portion is not excessively strong, and therefore it is possible to peel off the locking portion easily while suppressing the tearing of the skin-side sheet when removing the diaper from the wearer's body.

In such a underpants-shaped disposable diaper, it is desirable that in at least one of the front waist portion and the back waist portion, a number of overlaid sheet members that are overlaid in a thickness direction in a certain region in the vertical direction is different from a number of overlaid sheet members that are overlaid in the thickness direction in another region in the vertical direction.

According to the underpants-shaped disposable diaper, the peeling strength when peeling off the locking portion along the vertical direction can be adjusted for each of the regions. Accordingly, in a portion of the waist portion that is likely to be torn in the lateral direction, adjustment can be made such as decreasing the peeling strength of the locking portion.

In such a underpants-shaped disposable diaper, it is desirable that in at least one of the front waist portion and the back waist portion, the locking portion has a different locking pattern for each of regions in which the number of overlaid sheet members overlaid in the thickness direction is different.

According to the underpants-shaped disposable diaper, by varying the locking pattern for each of the regions in the waist portion in which the number of sheets overlaid in the thickness direction is different, it is possible to suppress the locking strength from becoming excessively strong or weak depending on the regions in which the number of overlaid sheets is different.

In such a underpants-shaped disposable diaper, it is desirable that in at least one of the front waist portion and the back waist portion, in the vertical direction, a position where the locking pattern changes is different from a position where the number of overlaid sheet members that are overlaid in the thickness direction changes.

According to the underpants-shaped disposable diaper, the position where the number of overlaid sheet members in the waist portion changes and the position where the locking strength of the locking portion changes are shifted. This makes the force that acts in a case of peeling the locking portion off change gradually. That is, the sudden change of the force acting on the waist portion at the time of peeling off the locking portion is suppressed, making it less likely to tear the waist portion.

In such a underpants-shaped disposable diaper, it is desirable that at least one of the front waist portion and the back waist portion includes: a first region having a larger number of overlaid sheet members that are overlaid in the thickness direction; and a second region having a smaller number of overlaid sheet members that re overlaid in the thickness direction, than in the first region, and a peeling strength of the locking portion in the first region is weaker than a peeling strength of the locking portion in the second region.

According to the underpants-shaped disposable diaper, in the first region where a larger number of sheet members are overlaid, the locking portion is more likely to be peeled off compared to the second region where a smaller number of sheets are overlaid. This makes it possible to reduce the force acting on the skin-side sheet member in the first region. This makes it less likely to tear the skin-side sheet member.

In such a underpants-shaped disposable diaper, it is desirable that in the region adjacent to inside of the locking portion in the lateral direction, the skin-side sheet member and the sheet member overlaid on the non-skin side of the skin-side sheet member include a joining portion where the skin-side sheet member and the sheet member are joined.

According to the underpants-shaped disposable diaper, in the adjacent region, the skin-side sheet member and the sheet member adjacent to the non-skin side thereof are at least partially joined in the thickness direction. Accordingly, the material strength of the waist portions increases, making it easier to suppress tearing in the lateral direction.

### Embodiment

Hereinafter, an embodiment of an absorbent article according to the present invention will be described by way of example of a underpants-shaped disposable diaper for adults (hereinafter also referred to as a "diaper 1"). It should be noted that the absorbent article according to the present invention is applicable to an underpants-shaped disposable diapers for infants or others.

### Configuration of Diaper 1

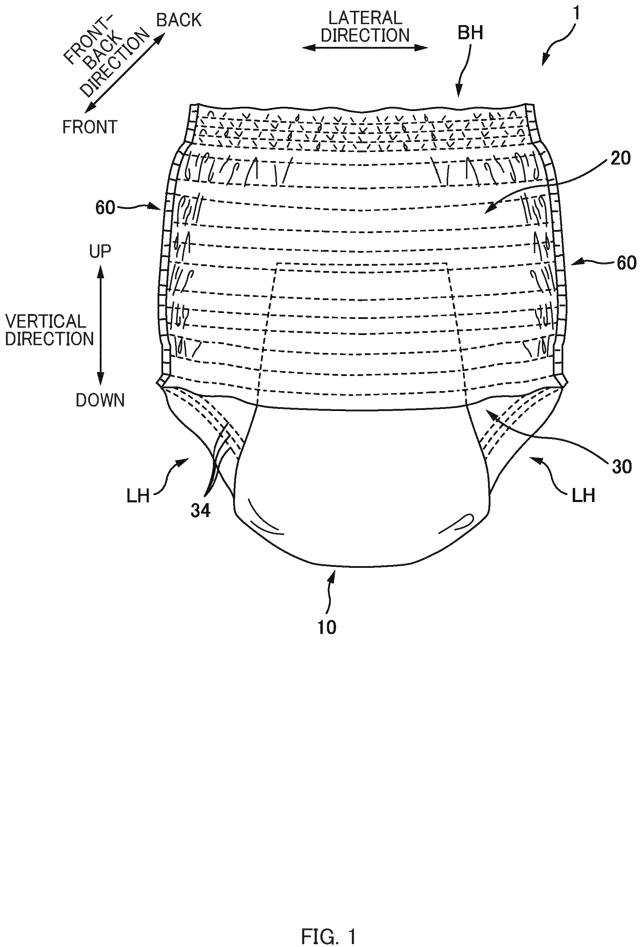

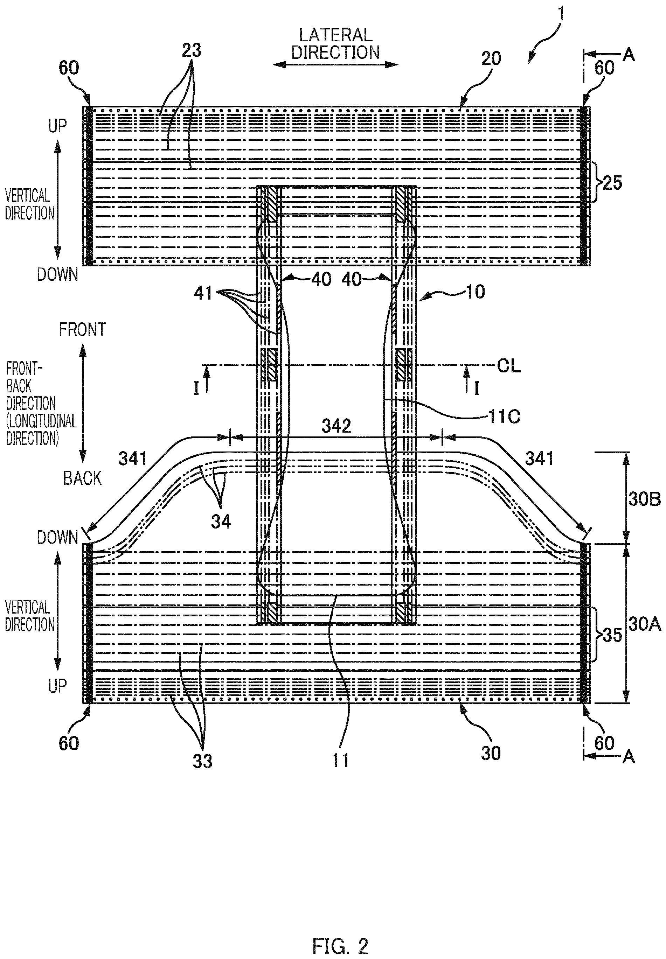

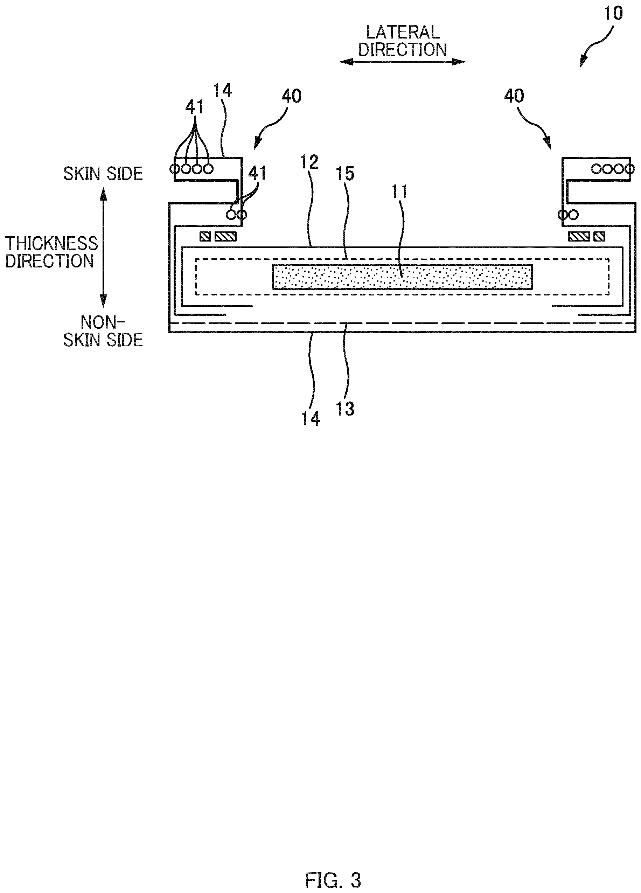

FIG. 1 is a schematic front view of the diaper 1. FIG. 2 is a schematic plan view of the diaper 1 in an unfolded and stretched state. FIG. 3 is a schematic cross-sectional view taken along a line I-I in FIG. 2.

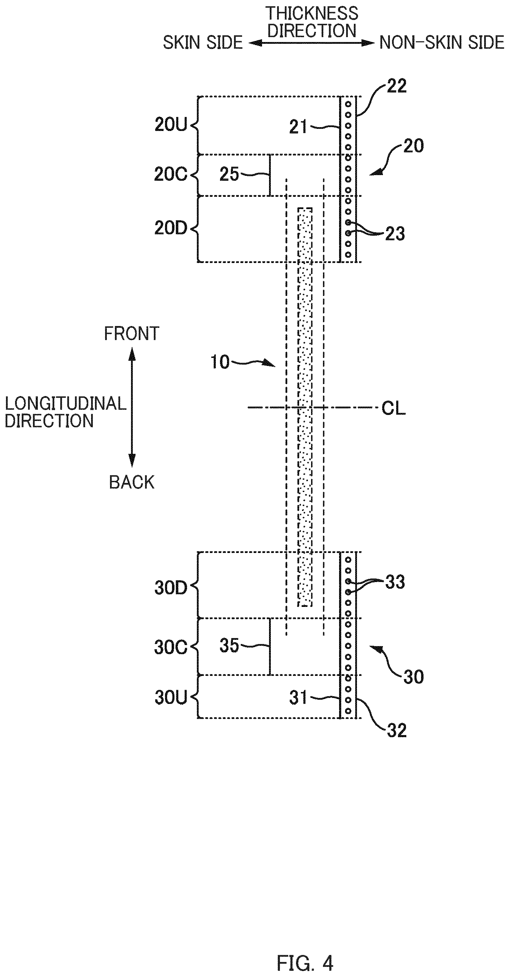

FIG. 4 is a schematic cross-sectional view taken along a line A-A in FIG. 2. It should be noted that the "stretched state" of the diaper 1 refers to a state in which the entire diaper 1 (the entire product) has been stretched to eliminate wrinkles, or more specifically, a state in which the diaper has been stretched until the dimensions of constituent members of the diaper 1 (e.g., an absorbent body 10, waist portions 20 and 30, or the like that will be described later) match or are close to the dimensions of the members on their own.

In the underpants-shaped state shown in FIG. 1, the diaper 1 has a vertical direction, a lateral direction, and a front-back direction that intersect with each other and has a waist opening BH and a pair of leg openings LH. The upper side in the vertical direction corresponds to the waist opening BH side, and the lower side corresponds to the crotch side. In addition, the front side in the front-back direction corresponds to the wearer's stomach side, and the back side corresponds to the wearer's back side. In addition, in the unfolded state of FIG. 2, the diaper 1 has the front-back direction and the lateral direction that intersect each other. The front-back direction corresponds to the longitudinal direction of the absorbent body 10. In addition, as shown in FIGS. 3 and 4, a direction in which materials for configuring the diaper 1 are overlaid is referred to as a thickness direction. In the thickness direction, the side that comes into contact with the wearer is referred to as the skin side, and the side opposite to the skin side is referred to as the non-skin side.

The diaper 1 has an absorbent main body 10, a front waist portion 20, and a back waist portion 30. In the diaper 1 in an underpants-shaped state, the absorbent main body 10 (later-described absorbent core 11) is arranged extending along the vertical direction, and is folded in front-back direction in the vertically lower end portion. In addition, the front waist portion 20 is joined to the front upper end portion of the absorbent body 10 from the non-skin side, and the back waist portion 30 is joined to the back upper end portion of the absorbent body 10 from the non-skin side.

When the diaper 1 is in the unfolded state of FIG. 2, the front waist portion 20 and the back waist portion 30 are arranged such that the longitudinal directions thereof conform to the lateral direction of the diaper 1. In addition, the longitudinal one-side (front) end portion of the absorbent body 10 is arranged in the lateral central portion of the front waist portion 20, and the longitudinal other-side (back) end portion of the absorbent body 10 is arranged in the lateral central portion of the back waist portion 30. When the diaper 1 is in the unfolded state shown in FIG. 2, the absorbent main body 10 is folded one time at substantially the center position CL in the front-back direction (longitudinal direction). The two lateral side portions of the front waist portion 20 are respectively locked with two lateral side portions of the back waist portion 30 by a pair of locking portions 60 and 60, thereby forming the diaper 1 into an underpants shape as in FIG. 1. In the present embodiment, the locking portions 60 are formed by a joining means such as welding. The locking portions 60 will be described later in detail.

The absorbent body 10 includes: the absorbent core 11 that absorbs excrement; a hydrophilic skin-side sheet 12 arranged on the wearer's skin side of the absorbent core 11; a liquid-impermeable non-skin-side sheet 13 arranged on the non-skin side of the absorbent core 11; and a sheet member 14. The two lateral side portions of the skin-side sheet 12 are folded back to the non-skin side so as to wrap the absorbent core 11. The sheet member 14 is arranged on the non-skin side with respect to the non-skin-side sheet 13, and forms the leak-proof wall portions 40 on the skin side with respect to the skin-side sheet 12.

The absorbent core 11 is made by shaping liquid absorbent fibers **(e.g.,** pulp fibers) containing a superabsorbent polymer (SAP), into a predetermined shape. In addition, the absorbent core 11 may be covered with a liquid-permeable core-wrapping sheet 15.

In addition, the absorbent core 11 of the present embodiment includes a narrow portion 11C in the central portion in the front-back direction, and the narrow portion 11C is a portion where the lateral length of the absorbent core 11 is shorter (has a narrower width) compared with the front end portion and the back end portion. Accordingly, the absorbent core 11 has a substantially hourglass shape in a plan view as shown in FIG. **2****.** This narrow portion 11C is a portion that is sandwiched between the wearer's legs while the diaper 1 is put on, and the absorbent core 11 more easily fits to the wearer's crotch due to the decreased lateral length (decreased width) of this portion.

A pair of the leak-proof wall portions 40 are respectively provided on two lateral side portions of the absorbent body 10, extending along the front-back direction (the longitudinal direction of the absorbent body 10). In the present embodiment, the pair of leak-proof wall portions 40 are each formed by folding the sheet member 14 that constitutes the exterior of the absorbent main body 10 at a plurality of positions as shown in FIG. 3. In addition, in the leak-proof wall portion 40, a plurality of leak-proof-wall stretchable members 41 such as an elastic string and the like are fixed in a state of being stretched in the front-back direction of the absorbent main body 10 (corresponding to the vertical direction of the diaper 1). While the diaper 1 is put on, the leak-proof wall portion 40 contracts along the front-back direction of the absorbent main body 10 and rises to the skin side of the wearer due to the stretchability exhibited by the leak-proof-wall stretchable member 41, thereby fitting to the wearer's crotch portion.

The front waist portion 20 includes: an inner-layer sheet 21; an outer-layer sheet 22 which is arranged on the non-skin side with respect to the inner-layer sheet 21; waist elastic members 23; and a skin surface sheet 25 which is arranged on the skin side of the inner-layer sheet 21. The inner-layer sheet 21 and the outer-layer sheet 22 are sheet members having a rectangular shape in a plan view as shown in FIG. 2, and are made of SMS nonwoven fabric or the like. The waist elastic members 23 are arranged side-by-side in the vertical direction between the inner-layer sheet 21 and the outer-layer sheet 22, and are fixed in a state of being stretched in the lateral direction. The front waist portion 20 fits to the wearer's waist due to the stretchability exhibited by the waist elastic members 23. In FIG. 4, the skin surface sheet 25 is a sheet member which is arranged so as to cover the upper end portion of the absorbent main body 10 from the skin side, and functions as a cover sheet. Accordingly, it is possible to suppress the upper end edge of the absorbent main body 10 from digging into the wearer's waist portion while the diaper 1 is put on. The skin surface sheet 25 is made of SMS nonwoven fabric or the like.

It should be noted that, in the diaper 1, the skin surface sheet 25 is provided so as to be continuous from one end to the other end in the lateral direction, and arranged so as to overlap the locking portion 60 in two end portions in the lateral direction. That is, the skin surface sheet 25 together with the inner-layer sheet 21 and the outer-layer sheet 22 are locked to the back waist portion 30 by the locking portion 60. However, the skin surface sheet 25 may be provided only in the central region in the lateral direction and arranged so as not to overlap the locking portion 60. In addition, the diaper 1 does not necessarily have to include the skin surface sheet 25.

In addition, as shown in FIG. 4, in the front waist portion 20, a region on an upper side with respect to the upper end of the skin surface sheet 25 in the vertical direction is defined as 20U (upper end region), a region which overlaps the skin surface sheet 25 is defined as 20C (central region), and a region on a lower side with respect to the lower end of the skin surface sheet 25 is defined as 20D (lower end region). In the front waist portion 20, the sheet member which is arranged the farthest on the skin side in the thickness direction is referred to as a "skin-side sheet member". In the case of the front waist portion 20 shown in FIG. 4, in the upper end region 20U and the lower end region 20D, the inner-layer sheet 21 corresponds to the skin-side sheet member, and, in the central region 20C, the skin surface sheet 25 corresponds to the skin-side sheet member.

The back waist portion 30 has substantially the same function and structure as the front waist portion 20. That is, the back waist portion 30 includes: an inner-layer sheet 31; an outer-layer sheet 32 which is arranged on a non-skin side with respect to the inner-layer sheet 31; waist elastic members 33, and a skin surface sheet 35 (see FIG. 4).

In addition, in the back waist portion 30, a region on an upper side with respect to the upper end of the skin surface sheet 35 in the vertical direction is defined as 30U (upper end region), a region which overlaps the skin surface sheet 35 is defined as 30C (central region), and a region on a lower side with respect to the lower end of the skin surface sheet 35 is defined as 30D (lower end region). In the case of the back waist portion 30 of the diaper 1, in the upper end region 30U and the lower end region 30D, the inner-layer sheet 31 corresponds to the skin-side sheet member, and, in the central region 30C, the skin surface sheet 35 corresponds to the skin-side sheet member.

In addition, in the unfolded and stretched state of FIG. 2, the back waist portion 30 includes an overlapping portion 30A which is to overlap the front waist portion 20 in the front-back direction, and an extension portion 30B which is to extend downward beyond the front waist portion 20. The overlapping portion 30A has a rectangular shape in a plan view, and the extension portion 30B has an inverted trapezoidal shape in a plan view. The wearer's buttocks can be covered with the extension portion 30B. On the other hand, the front waist portion 20 has a shorter vertical length than the back waist portion 30, and does not extend to the vicinity of the wearer's crotch. For this reason, leg movement is not hindered when the wearer lifts their legs toward their stomach while walking for example, making it easier for the wearer to walk.

Extension-portion elastic members 34 are provided extending along the lower end of the extension portion 30B of the back waist portion 30. Specifically, the extension-portion elastic members 34 each include a pair of inclined portions 341 which are inclined downward and laterally inward, and a straight portion 342 which extends along the lateral direction between the pair of inclined portions 341. Similarly to the waist elastic members 33, the extension-portion elastic members 34 are fixed in a stretched state between the inner-layer sheet 31 and the outer-layer sheet 32. The extension-portion elastic members 34 make the extension portion 30B fit to the wearer's buttocks, suppressing the extension portion 30B being curled up.

However, the present invention is not limited to the above configuration. For example, a configuration is possible in which the front waist portion 20 has the same shape as the back waist portion 30 in a plan view, and includes the elastic members similar to the extension-portion elastic members 34 of the back waist portion 30. Specifically, the front waist portion 20 may include inclined elastic members that are inclined downward and laterally inward in the lower end portion. Conversely, a configuration is possible in which the back waist portion 30 has the same rectangular shape as the front waist portion 20 in a plan view, and does not include the extension-portion elastic members 34.

### Removing operation of diaper 1

An operation of removing the diaper 1 after excresion (after use), from the wearer's body during usage of the diaper 1 will be described.

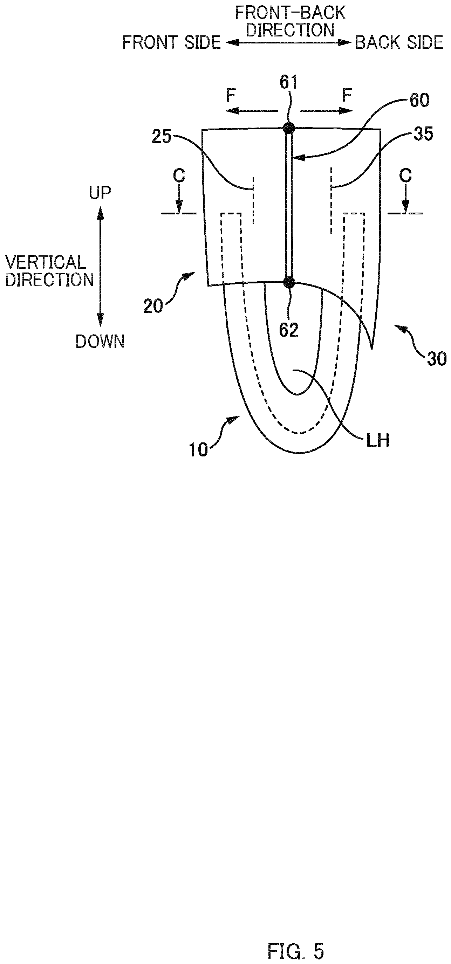

FIG. 5 is a schematic view showing the diaper 1 in the underpants-shaped state when seen from one side in the lateral direction (right side in FIG. 5). At the time of removing the underpants-shaped disposable diaper such as the diaper 1 from the wearer's body, the following method is generally used: peeling off the locking portions 60 which lock the waist portions 20 and 30 in two lateral end portions of the front waist portion 20 and two lateral end portions of the back waist portion 30, and separating the front waist portion 20 and the back waist portion 30. That is, the diaper 1 is removed from the wearer's body by peeling the lockings of the front waist portion 20 and the back waist portion 30, which form in an annular shape along the wearer's waist, and unfolding the diaper 1 as shown in FIG. 2.

The locking portions 60 can be peeled off by pulling the front waist portion 20 and the back waist portion 30 in opposite directions. In FIG. 5, a user pulls the front waist portion 20 forward (toward stomach side) in the front-back direction, and pulls the back waist portion 30 backward (toward back side) in the front-back direction, making it possible to peel off the locking portions 60. At this time, it is recommended that, by grabbing upper end portions (or lower end portions) of the waist portions 20 and 30 and pulling away the grabbed portions from each other in the front-back direction with a predetermined tensile force F, it makes the locking portion 60 be peeled along the vertical direction from the upper end 61 (or lower end 62) of the locking portion 60.

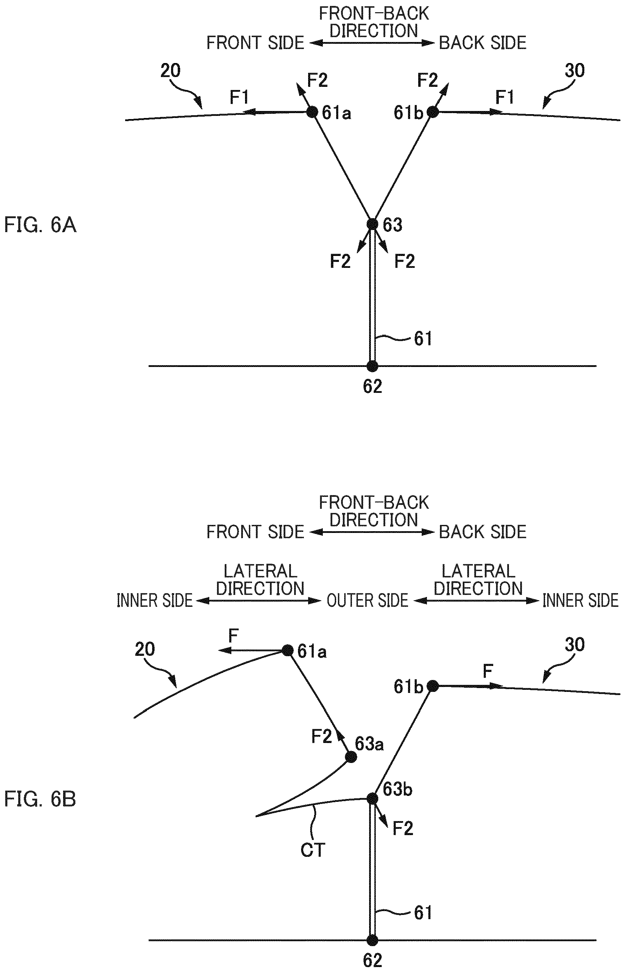

FIGS. 6A and 6B are schematic diagrams illustrating the peeling of the locking portion 60. Similarly to FIG. 5, FIGS. 6A and 6B show a state where the locking portion 60 of the diaper 1 is seen from one side (right side) in the lateral direction.

FIG. 6A shows a force that acts on the front waist portion 20 and the back waist portion 30 when peeling off the locking portion 60 from the upper end 61 toward the lower end 62 in the vertical direction. As described above, in a case where the user grabs the upper end portions of the front waist portion 20 and the back waist portion 30 and pulls away the grabbed portion from each other in the front-back direction with a tensile force F1, the upper end 61 of the locking portion 60 is peeled off at the timing when the tensile force F1 becomes larger than the peeling strength of the locking portion 60, separating the upper end 61a of the front waist portion 20 and the upper end 61b of the back waist portion 30. If continuously keeping pulling the waist portions 20 and 30 in the front-back direction, the locking portion 60 is peeled off in order from the upper side (upper ends 61a and 61b) to the lower side (lower end 62) in the vertical direction. That is, a peeling point 63 at which the locking portion 60 is being peeled off moves along the locking portion 60 from the upper side to the lower side. It should be noted that, in the case of peeling off the locking portion 60, the locking portion 60 may be peeled off from the lower side (lower end 62) to the upper side (upper end 61).

At this time, at locations between the upper end 61a and the peeling point 63 in the front waist portion 20, tension F2 as shown in FIG. 6A acts due to the tensile force F1. Similarly, at locations between the upper end 61b and the peeling point 63 in the back waist portion 30, the tension F2 acts.

In addition, in the case where the tension F2 becomes equal to or greater than a predetermined magnitude, there is a risk that a part of the sheet member constituting the waist portions 20 and 30 is torn at any position between the upper end 61b and the peeling point 63. FIG. 6B shows a case where a part of the front waist portion 20 is torn in the peeling operation of the locking portion 60 described in FIG. 6A. Specifically, in FIG. 6B, the tension F2 becomes greater than the tearing strength at the time of tearing the sheet member constituting the front waist portion 20, and this causes breakage at the peeling point 63, separating the peeling point into 63a and 63b. Thereby, the front waist portion 20 is torn along the lateral direction, forming a tear CT.

In the case where such a tear is formed in the waist portions 20 and 30, it makes it difficult to further peel off the locking portions 60, and there is a risk that the removing operation of the diaper 1 is obstructed. In addition, in the case where the tear is large, there is a risk that the excrement such as feces adhering to the inside of the diaper 1 (absorbent main body 10) leaks to the outside.

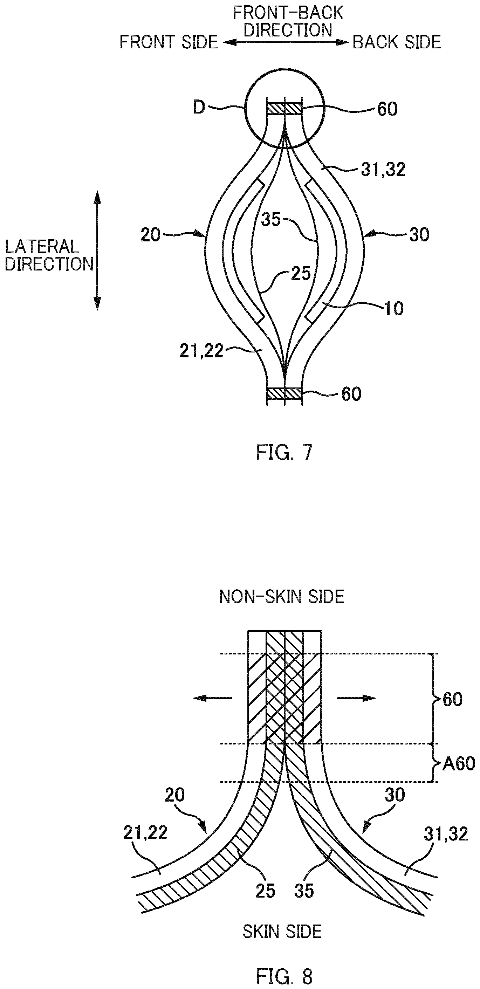

Next, among a plurality of sheet members constituting the waist portions 20 and 30, a sheet member that is likely to be torn at the time of peeling off the locking portion 60 will be described. FIG. 7 is a schematic cross-sectional view taken along a line C-C in FIG. 5. FIG. 8 is a schematic enlarged view of a region D (locking portion 60) in FIG. 7.

As shown in FIGS. 7 and 8, in the locking portions 60, the followings sheets are joined (locked) with being overlaid in the thickness direction: the inner-layer sheet 21, the outer-layer sheet 22, and the skin surface sheet 25 which constitute the front waist portion 20; and the inner-layer sheet 31, the outer-layer sheet 32, and the skin surface sheet 35 which constitute the back waist portion 30. In the case of peeling off the locking portion 60, among the sheet members of the front waist portion 20 and the back waist portion 30, it is necessary to peel off the sheet members whose located-the-farthest-on-the-skin-side surface are in direct contact with each other, in the locking portion 60. In the example of FIG. 7 and FIG. 8, in the locking portion 60, the skin surface sheet 25 is arranged as the skin-side sheet member that is located the farthest on the skin side in the front waist portion 20, and the skin surface sheet 35 is arranged as the skin-side sheet member that is located the farthest on the skin side in the back waist portion 30. Accordingly, in the case shown in FIGS. 7 and 8, in order to peel off the locking portion 60, it is necessary to peel off a joining surface (interface) which joins the skin surface sheet 25 of the front waist portion 20 and the skin surface sheet 35 of the back waist portion 30.

In FIG. 8, in a case where the front waist portion 20 and the back waist portion 30 are each pulled away from each other in the front-back direction, a force acts in a direction in which the joining surfaces (interfaces) of the skin-side sheet member (skin surface sheet 25), on the front side and the skin-side sheet member (skin surface sheet 35) on the back side are peeled off. As a result, in the locking portion 60 shown with hatched lines in FIG. 8, the front waist portion 20 and the back waist portion 30 are peeled off.

Here, the skin surface sheet 25 and the skin surface sheet 35, which are the skin-side sheet members, are joined to the corresponding sheet members that are respectively overlaid on the non-skin sides of the sheet 25 and 35, with an adhesive such as a hot-melt adhesive. Specifically, the skin surface sheet 25 is joined to the inner-layer sheet 21 (outer-layer sheet 22), and the skin surface sheet 35 is joined to the inner-layer sheet 31 (outer-layer sheet 32) (see FIGS. 4 and 8). The adhesive is not uniformly applied to the surface of the sheet member, and is intermittently applied, for example, by spiral coating or Ω-shaped coating (not shown). Accordingly, the skin-side sheet members (the skin surface sheet 25 and the skin surface sheet 35) each include: a joining portion where the skin-side sheet member is joined to the sheet member that is overlaid on the non-skin side thereof; and a non-joining portion that is not joined.

In such a non-joining portion, at the time of peeling off the locking portions 60, a force acts so as to pull off the skin surface sheet 25 (skin surface sheet 35) from the inner-layer sheet 21 (inner-layer sheet 31). The tensile force for peeling off the locking portion 60 of the waist portions 20 and 30 is more likely to most strongly act on the skin-side sheet member (skin surface sheets 25 and 35) arranged the farthest on the skin side. In the case where the joining portion is formed in the entire region of the interface between the skin surface sheet 25 (35) and the inner-layer sheet 21 (31), the force for peeling off the skin surface sheet 25 (35) acts on the skin surface sheet 25 (35) and the inner-layer sheet 21 (31) as a unit. On the other hand, in the case where there is a non-joining portion at the interface between the skin surface sheet 25 (35) and the inner-layer sheet 21 (31) as in the present embodiment, the unity of the skin surface sheet 25 (35) and the inner-layer sheet 21 (31) is lost, and accordingly, a force for peeling off the interface between the skin surface sheets 25 and 35 greatly acts on the skin surface sheet 25 (35). Therefore, the skin surface sheet 25 (35) which has received a large force is likely to be torn.

Particularly, an adjacent region A60 which is adjacent to inside of the locking portion 60 in the lateral direction includes a non-joining portion where the skin surface sheet 25 (35) and the inner-layer sheet 21 (32) are not joined. In adjacent region A60, the front waist portion 20 and the back waist portion 30 are not joined to each other (see FIG. 8), and accordingly, the tensile force for peeling off the locking portion 60 is likely to act directly on the skin surface sheets 25 and 35. As described above, there is a risk that the skin surface sheets 25 and 35 (skin-side sheet members) are torn along the lateral direction when peeling off the locking portions 60.

In contrast, in the diaper 1 of the present embodiment, the tearing strength of the skin-side sheet member (skin surface sheet 25 and 35) which is arranged the farthest on the skin side of the waist portion 20 (30), at the time of tearing it along the lateral direction, becomes greater than the peeling strength of the locking portion 60 at the time of peeling it off. This prevents the skin-side sheet member from being torn at the time of peeling apart the locking portion 60.

In this case, increasing the basis weight of the skin-side sheet member can increase the tearing strength of the skin-side sheet member. However, since the skin-side sheet member is a member having a high possibility of coming into direct contact with the wearer's body while the diaper 1 is put on, there is a risk that wearing comfort to wear and breathability of the diaper 1 deteriorates in the case of using the sheet member having an excessively high basis weight. In addition, there is a risk that the manufacturing cost (material cost) of the diaper 1 increases. Therefore, in the diaper 1 of the present embodiment, the basis weight of the skin-side sheet member is set to be equal to or lower than a predetermined value. Specifically, a nonwoven fabric sheet or the like is used in which a total value of the basis weights of the skin-side sheet member and the sheet member located the farthest on the non-skin side is 32 g/m² or less. This makes it possible to suppress the increase in manufacturing cost and the deterioration of breathability.

It should be noted that, in the adjacent region A60 of FIG. 8, the skin-side sheet member (skin surface sheet 25) and the sheet member adjacent to the non-skin side thereof (inner-layer sheet 21) have a joining portion where they are at least partially joined to each other. That is, the skin-side sheet member and the sheet member adjacent to the non-skin side thereof are joined in a part of the region, in a state of being overlaid in the thickness direction. Accordingly, the material strength of the waist portion 20 (30) increases, making it easier to suppress the tearing in the lateral direction. In addition, when peeling off the locking portion 60, it is possible to suppress peeling and separating apart into pieces the sheet members that are overlaid in the waist portion 20 (in FIG. 8, the skin surface sheet 25 and the inner-layer sheet 21).

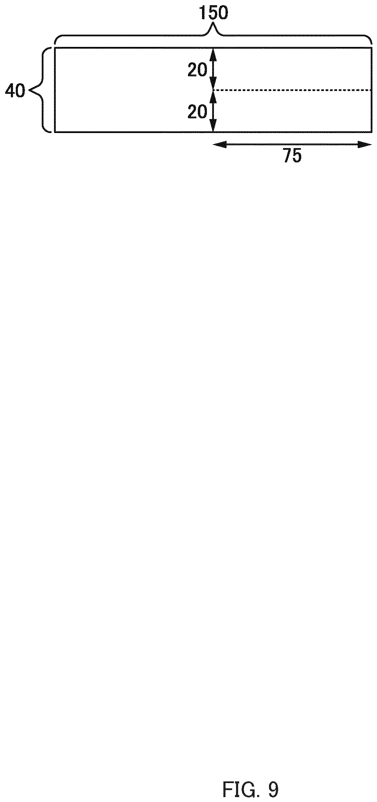

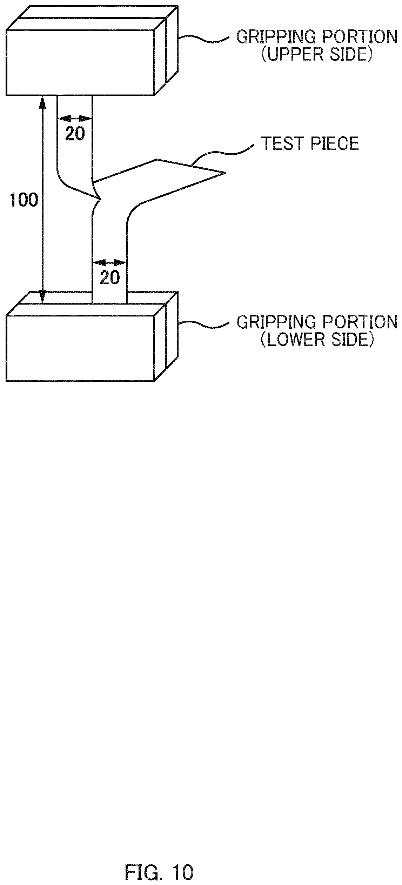

Next, a method for measuring the tearing strength of the skin-side sheet member and the peeling strength of the locking portions 60 will be described. The tearing strength of the skin-side sheet member can be measured as follows. FIG. 9 is a diagram illustrating a test piece used for measuring the tearing strength of the skin-side sheet member. FIG. 10 is a conceptual diagram illustrating a method for measuring the tearing strength of the skin-side sheet member.

In the measurement of the tearing strength of the skin-side sheet member, firstly, taking test pieces shown in FIG. 9 from the skin-side sheet member to be measured (for example, the skin surface sheets 25 and 35) is performed. First, in the vicinity of the locking portion 60 of the diaper 1, the front waist portion 20 (back waist portion 30) is cut off, and the overlaid sheet members are separated into individual sheets by being immersed in toluene or the like. Then, a skin-side sheet member, which is a target, is collected. From the collected skin-side sheet member, five rectangular sheets are cut out to have a long side of 150 mm or more and a short side of 40 mm or more. At this time, a long-side direction of the sheet is set to a direction extending along the lateral direction of the diaper 1. Then, for each of the cut sheets, a notch having a length of 75 mm is made from the center in a short-side direction to obtain a test piece (see FIG. 9). The portion where the notch is provided serves as a gripping margin.

Subsequently, a tensile test is performed on the test pieces using a commercially available constant-speed extension-type tensile tester (e.g., a tensile tester manufactured by Shimadzu Corporation: Model No. AG-1kNI). As shown in FIG. 10, a pair of gripping portions provided in the tensile tester are arranged apart from each other at a distance of 100 mm vertically, and each test piece is fixed so as not to loosen by making the gripping margins of the test piece each be sandwiched. In this state, the test piece is pulled in the vertical direction at a speed of 200 mm/min, a magnitude of a force at the time when the test piece starts to tear is recorded as a maximum strength. The average value of measurements of the five test pieces is taken as the tearing strength of the skin-side sheet member.

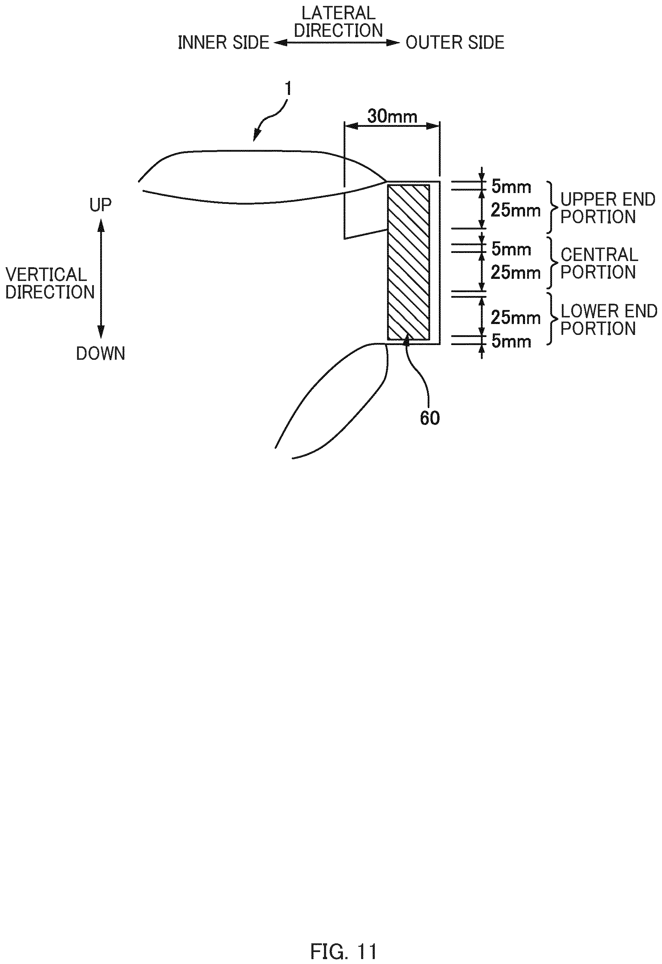

In addition, the peeling strength of the locking portion 60 can be measured as follows. FIG. 11 is a diagram illustrating a test piece used for measuring the peeling strength of the locking portion 60. In the measurement of the peeling strength of the locking portions 60, as shown in FIG. 11, sheet pieces having a length of 30 mm or more in the vertical direction and 30 mm or more in the lateral direction are cut out from each of the regions obtained by dividing the locking portion 60 of the diaper 1 into three equal parts in the vertical direction (an upper end portion, a central portion, and a lower end portion). Then, the sheet pieces are taken as test pieces. However, in the case where the number of sheet members overlaid in the thickness direction in the locking portion 60 is different as in the diaper 1, test pieces are prepared for each of the regions where the number of layers of the sheet members is different. In the diaper 1 of the present embodiment, five test pieces are prepared for each of the regions 20U, 20C, and 20D (30U, 30C, and 30D) shown in FIG. 4.

Next, a gripping margin is produced for each test piece by peeling off the locking portion 60 from the upper end (or lower end) by approximately 5 mm in the vertical direction. Next, a front gripping margin of the test piece is made sandwiched in the gripping portion on one side of the same tensile tester as that described in FIG. 10, and a back gripping margin of the test piece is made sandwiched in the gripping portion on the other side of the tensile tester. It should be noted that measuring can be made easier by splicing the gripping margin of the test piece and a gummed tape or the like having a length longer than that of the gripping margin, to extend the gripping margin. Then, by setting a space between the pair of gripping portions to 5 mm, the test piece is fixed so as not to loosen. In this state, the test piece is pulled in the vertical direction at a speed of 300 mm/min, a magnitude of a force at the time when the locking portion 60 has been completely peeled off is recorded as a maximum strength. The average value of measurements for the five test pieces is taken as the peeling strength of the locking portion 60.

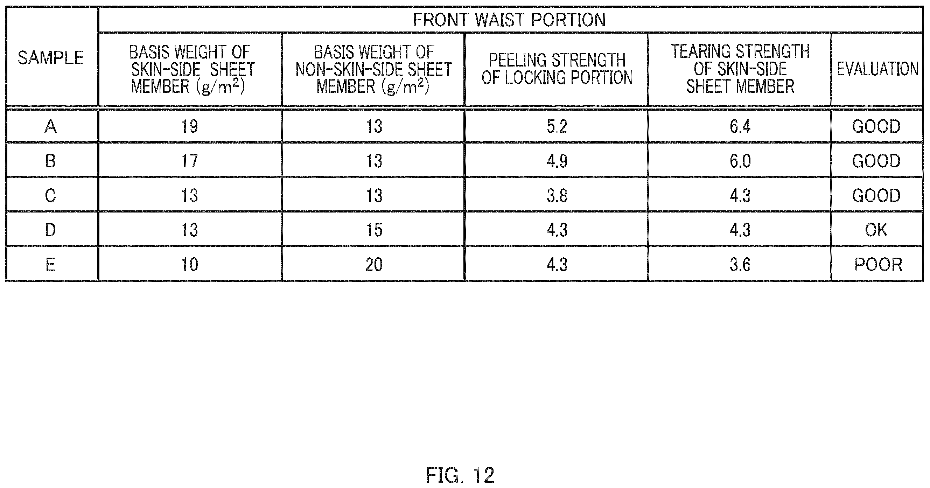

FIG. 12 is a table showing results of the measurement. FIG. 12 shows results obtained for the front waist portion 20 by the following manner: five types of diaper were prepared as samples A to E in which the basis weight of the skin-side sheet member arranged the farthest on the skin side in the thickness direction is different and in which the basis weight of the sheet member located the farthest on the non-skin side in the thickness direction is different; and the tearing strength of the skin-side sheet member in the lateral direction and the peeling strength of the locking portion 60 were measured for each of the samples by the method described above. The operation of actually peeling the locking portion 60 was performed a plurality of times for each sample. A case where no tearing of the sheet member in the lateral direction occurred was evaluated as GOOD, a case where the tearing of the sheet member inevitably occurs was evaluated as POOR, and a case where the tearing occurs in some not all of the plurality of times of operations was evaluated as OK.

It should be noted that the "basis weight" is a weight per unit area of the sheet member to be measured. For example, the basis weight can be determined as follow: sheet pieces having a predetermined size are cut out at a plurality of locations **(e.g.,** ten locations) from the skin-side sheet member 20; the weight of each sheet piece is measured using an electronic balance or the like; and the average value of values obtained by dividing the weights of the sheet pieces by the area of the sheet pieces, to be the basis weight.

As a result of the measurement, in the front waist portion 20 where the sum of the basis weight of the sheet member (skin-side sheet member) arranged the farthest on the skin side and the basis weight of the sheet member arranged the farthest on the non-skin side was 32 g/m² or less, in the case where the tearing strength in the lateral direction was greater than the peeling strength of the locking portions 60 (in the case of samples A to C in FIG. 12), the locking portion 60 could be peeled off without causing any lateral tearing of the sheet member. Therefore, it has been clarified that, in the underpants-shaped disposable diaper, by satisfying such conditions, it is possible to suppress the tearing of the material constituting the waist portion in the lateral direction when peeling the locking portion between the front waist portion and the back waist portion.

It should be noted that, though FIG. 12 shows the results of measurement for the front waist portion 20, but a sheet member having the same basis weight as that of the front waist portion 20 can be used also for the back waist portion 30. For the back waist portion 30 of each sample measured in FIG. 12, the same sheet member having the same basis weight as that of the front waist portion 20 was used. In addition, even if materials having different basis weights are used for the front waist portion 20 and the back waist portion 30, the diaper 1 of the present embodiment is applicable as long as the relationship described above is satisfied. That is, it is sufficient that the sum of the basis weight of the sheet member arranged the farthest on the skin side (skin-side sheet member) and the basis weight of the sheet member arranged the farthest on the non-skin side is 32 g/m² or less, and that the tearing strength in the lateral direction is greater than the peeling strength of the locking portions 60.

In addition, from the results of FIG. 12, it has been clarified that, in the case where the basis weights of the skin-side sheet members arranged the farthest on the skin side of the waist portions 20 and 30 is equal to or more than the basis weights of the sheet members arranged the farthest on the non-skin side, it is less likely to cause lateral tearing of the waist portions 20 and 30. That is, in FIG. 12, in samples A and B in which the basis weight of the skin-side sheet member is equal to or more than the basis weight of the non-skin-side sheet member, no lateral tearing of the waist portions 20 and 30 occurred. On the other hand, in samples C and D in which the basis weight of the skin-side sheet member is less than the basis weight of the non-skin-side sheet member, the lateral tear of the waist portions 20 and 30 occurred in some cases.

As described above, in the operation of peeling off the locking portion 60 of the waist portions 20 and 30, a great force is likely to act on the skin-side sheet member arranged the farthest on the skin side. That is, among the plurality of sheet members constituting the waist portions 20 and 30, the skin-side sheet member (for example, the skin surface sheets 25 and 35) is most likely to be torn. Accordingly, for the skin-side sheet member, it is desirable to use a sheet member having as a great basis weight as possible, within an applicable range (the sum of the basis weights of the skin-side sheet member and the non-skin-side sheet member is 32 g/m² or less). On the other hand, a force is less likely to act on the non-skin-side sheet member when peeling off the locking portion 60 than on the skin-side sheet member, and the non-skin-side sheet member is less likely to be torn. Therefore, a sheet member having a basis weight smaller than that of the skin-side sheet member can be used as the non-skin-side sheet member. As described above, in the waist portions 20 and 30, on the non-skin side with respect to the skin-side sheet members, it is possible to use a sheet member having a smaller basis weight than that of the skin-side sheet member. This makes it possible to reduce the material cost of the entire diaper 1.

Note that it is sufficient that the relationship described above is satisfied in at least one of the upper end region and the lower end region of the locking portion 60. In the case where the locking portion 60 is to be peeled off at the time of removing the diaper 1 from the wearer's body, either one of the following operations is performed: peeling off the locking portion 60 from the upper end 61 toward the lower end 62 of the locking portion 60; or peeling off the locking portion 60 from the lower end 62 toward the upper end 61 of the locking portion 60. That is, the upper end 61 or the lower end 62 serves as a peeling start point of the locking portion 60. Therefore, according to the use aspect of the diaper 1, it is sufficient that in at least one of the upper end region and the lower end region of the locking portion 60, the basis weight of the sheet member (skin-side sheet member) arranged the farthest on the skin side is equal to or more than the basis weight of the sheet member arranged the farthest on the non-skin side.

However, it is more preferable that, in both of the upper end region and the lower end region of the locking portion 60, the basis weight of the sheet member (skin-side sheet member) arranged the farthest on the skin side is equal to or more than the basis weight of the sheet member arranged the farthest on the non-skin side. With the configuration described above, it makes the tearing of the waist portions 20 and 30 easier to be suppressed in both cases of peeling off the locking portion 60 from the upper side and peeling off the locking portion 60 from the lower side. It should be noted that, in the diaper 1 of the present embodiment, in the locking portion 60, all of the sheet members arranged in the upper end regions (20U and 30U in FIG. 4) and the lower end regions (20D and 30D in FIG. 4) of the waist portions 20 and 30 are the same (inner-layer sheets 21 and 31). Accordingly, in both the upper end region and the lower end region of the locking portion 60, the basis weight of the skin-side sheet member (inner-layer sheets 21 and 31) can be equal to or more than the basis weight of the sheet member (outer-layer sheets 22 and 32) arranged the farthest on the non-skin side.

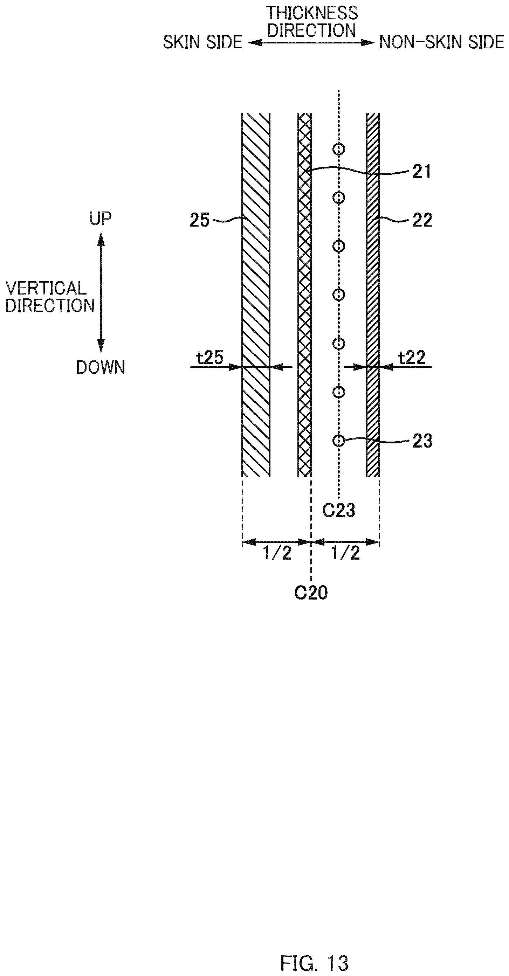

In addition, it is recommended that the arrangement of the waist elastic members 23 (33) provided in the waist portion 20 (30) is adjusted as follows. FIG. 13 is a schematic cross-sectional view showing how components of the front waist portion 20 are overlaid in the thickness direction. In the front waist portion 20, the skin surface sheet 25, the inner-layer sheet 21, the waist elastic members 23, and the outer-layer sheet 22 are overlaid in this order from the skin side toward the non-skin side in the thickness direction. In this state, it is desirable that the waist elastic members 23 are arranged on the non-skin side with respect to the central position of the front waist portion 20 in the thickness direction. In FIG. 13, in the thickness direction, the center c23 of the waist elastic members 23 is positioned on the non-skin side with respect to the center c20 of the front waist portion 20. According to the configuration described above, when the diaper 1 is put on, the distance between the waist elastic members 23 and the wearer's skin increases in the front waist portion 20, making it possible to suppress the waist elastic members 23 from tightly digging into the wearer's skin. That is, a contractive force generated by the waist elastic members 23 is less likely to directly act on the wearer's skin, making it possible to suppress the wearer feeling discomfort.

In addition, it is desirable that, among the sheet members constituting the waist portion 20 (30), the thickness of the skin-side sheet member arranged the farthest on the skin side is equal to or more than the thickness of the sheet member arranged the farthest on the non-skin side. In FIG. 13, the thickness t25 of the skin surface sheet 25, which serves as the skin-side sheet member, is equal to or more than the thickness t22 of the outer-layer sheet 22 arranged the farthest on the non-skin side (t25 ≥ t22). Increase in thickness of the skin-side sheet member can make it less likely to tear the skin-side sheet member at the time of peeling off the locking portions 60, while suppressing the tightening by the waist elastic members 23.

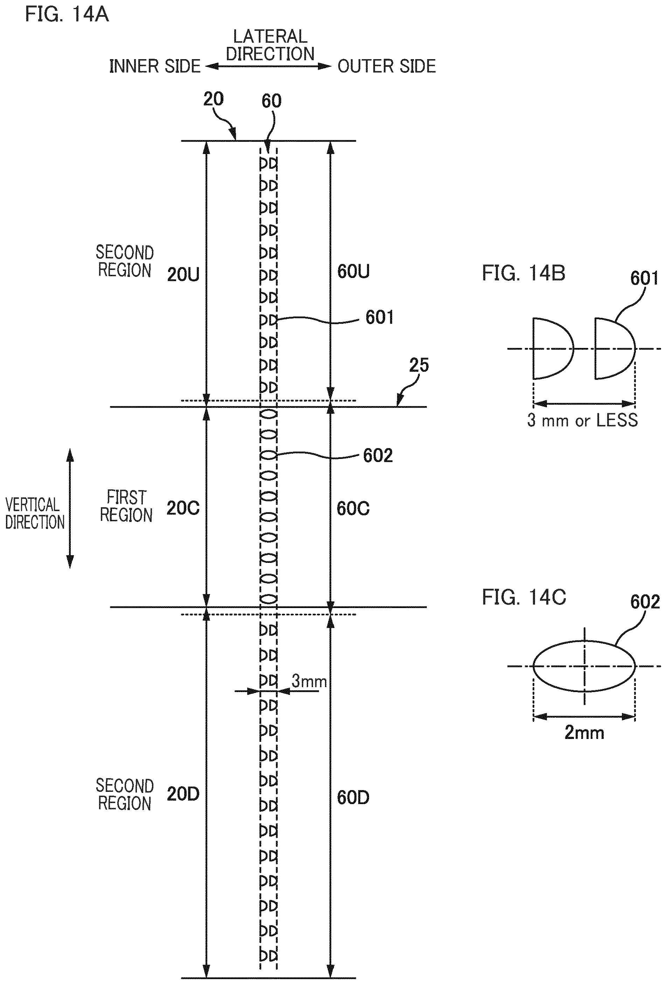

Next, a specific shape of the locking portion 60 will be described. FIGS. 14A to 14C are enlarged plan views illustrating the shape and arrangement of the locking portion 60. It should be noted that FIG. 14A shows the locking portion 60 provided at a right end in the lateral direction when the diaper 1 is seen from the front side in the front-back direction. As shown in FIG. 14A, the locking portion 60 of the diaper 1 is formed of two types of locking patterns. Specifically, the locking portion 60 is formed by arranging a plurality of first patterns 601 shown in FIG. 14B and second patterns 602 shown in FIG. 14C intermittently side-by-side along the vertical direction. The first pattern 601 is a locking pattern in which two semielliptical patterns are arranged side-by-side in the lateral direction, and the second pattern 602 is an elliptical locking pattern.

In the locking portion 60 of the diaper 1, as shown in FIGS. 14B and 14C, the width of the first pattern 601 and the width of the second pattern 602 in the lateral direction are both equal to or less than 3 mm. Also when the entire locking portion 60 is seen, the average value of the widths in the lateral direction is 3 mm or less (see FIG. 14A). It should be noted that the average value of the widths of the locking portion 60 in the lateral direction refers to the average value of the widths of all the locking patterns constituting the locking portion 60. For example, in a case of FIG. 14A, the average of the values of the widths that are measured in the lateral direction for all of the locking patterns (the first patterns 601 and the second patterns 602) included in the locking portion 60 is the average value of the widths of the locking portion 60.

In the case of locking the front waist portion 20 and the back waist portion 30 by the locking portion 60, as the total value of areas of the first patterns 601 and the second patterns 602 constituting the locking portion 60 increases, the strength (locking strength) of the locking portion 60 increases. However, if the locking strength of the locking portions 60 is excessively strong, there is a risk that the locking portions 60 cannot be peeled off when removing the diaper 1 from the wearer's body. In addition, as described above, if the peeling strength of the locking portions 60 is stronger than the tearing strength of the skin-side sheet members of the waist portions 20 and 30, there is a risk that the skin-side sheet member is torn. In contrast, in the diaper 1, by setting the width of the locking portions 60 in the lateral direction to 3 mm or less, the locking portion 60 can be easily peeled off during the operation of removing of the diaper 1. In addition, from experimental results in FIG. 12, in the case where the width of the locking portions 60 in the lateral direction is set to 3 mm or less, the tearing strength of the waist portions 20 and 30 can is stronger than the peeling strength of the locking portion 60. This enables to make the waist portions 20 and 30 difficult to be torn while peeling the locking portion 60 easily.

In addition, the waist portions 20 and 30 have regions in which the number of overlaid sheet members in the thickness direction is different. In other words, the number of overlaid sheet members that are overlaid in the thickness direction in a certain region in the vertical direction is different from the number of overlaid sheet members that are overlaid in the thickness direction in another region in the vertical direction. In FIG. 4, in the front waist portion 20, two sheet members are overlaid in the thickness direction in the upper end region 20U and the lower end region 20D, and three sheet members are overlaid in the thickness direction in the central region 20C. As described above, by providing the waist portions 20 and 30 with regions in which the number of overlaid sheet members is different, it makes it possible to adjust the peeling strength when peeling off the locking portion 60 along the vertical direction, for each of the regions. For example, in portions of the waist portions 20 and 30 that are likely to be torn in the lateral direction, decrease in peeling strength of the locking portion 60 makes it possible to smoothly peel off the locking portion 60 so as not to tear the waist portions 20 and 30.

It should be noted that, in the waist portions 20 and 30, regarding the locking portion 60, the locking strength in each of the regions can be appropriately adjusted by varying the locking pattern for each of the regions where the number of sheet members overlaid in the thickness direction is different. In FIG. 14A, the locking portion 60 in the upper end region 20U and the lower end region 20D of the waist portion 20 is formed of the first patterns 601, and the locking portion 60 in the central region 20C of the waist portion 20 is formed of the second patterns 602.

As shown in FIG. 4, in the upper end region 20U and the lower end region 20D of the waist portion 20, two sheet members are overlaid in the thickness direction, whereas, in the central region 20C, three sheet members are overlaid in the thickness direction. Accordingly, in the case where the locking portion 60 is formed in the same pattern (e.g., first patterns 601) in all the regions, there is a risk that the locking strength in the upper end region 20U and the lower end region 20D is excessive, or conversely, the locking strength in the central region 20C becomes weak. In contrast, in the present embodiment, by varying the locking pattern for each of the regions in which the number of sheets that are overlaid in the thickness direction of the waist portion 20 is different, it becomes less likely to cause unevenness in the locking strength across the regions. This makes possible to suppress the unintentional tearing of the locking portion 60 while the diaper 1 is put on, or to suppress that it makes it difficult to peel off the locking portion 60 when removing the diaper 1.

In addition, in the diaper 1, regarding the peeling strength of the locking portion 60, the patterns of the locking portion are adjusted so that the peeling strength in a region having a larger number of overlaid sheets in the thickness direction (defined as the first region, in FIG. 14A, the central region 20C) is less than the peeling strength in a region where a smaller number of overlaid sheets in the thickness direction (defined as the second region, in FIG. 14A, the upper end region 20U and the lower end region 20D). By making it easier to peel off the locking portion 60 in the region where a larger number of sheet members are overlaid (the first region, the central region 20C) compared to the region where a smaller number of sheet members are overlaid (the second region, the upper end region 20U and the lower end region 20D), it is possible to reduce the force acting on the skin-side sheet member (the skin surface sheet 25) in the first region (the central region 20C). This makes it less likely to tear the skin-side sheet member (the skin surface sheet 25).

It should be noted that, in the vertical direction, a position where the locking pattern changes and a position where the number of overlaid sheet members in the thickness direction of the waist portion 20 (30) changes may be shifted from each other. In FIG. 14A, letting a region in the locking portion 60 where the second patterns 602 are arranged be 60C, letting regions where the first patterns 601 are arranged be 60U and 60C, the boundary position in the vertical direction between the region 60U and the region 60C is a position different from the boundary position in the vertical direction between the upper end region 20U and the central region 20C of the waist portion 20 , for example. It should be noted that, in FIG. 14, the boundary position in the vertical direction between the region 60U and the region 60C refers to the central position between the lower end position of the first patterns 601 and the upper end position of the second patterns 602.

In the case where a position where the locking pattern changes and a position where the number of overlaid sheets of the waist portion 20 (30) changes are the same in the vertical direction, the number of overlaid sheet members of the waist portion 20 and the locking strength of the locking portion 60 simultaneously change in the changing position. Accordingly, the applied force suddenly changes when peeling off the locking portions 60 along the vertical direction, and this causes a risk that the waist portion 20 (skin-side sheet member) is easily torn. In contrast, in the diaper 1 of the present embodiment, since the position where the number of overlaid sheet members of the waist portion 20 changes and the position where the locking strength of the locking portions 60 changes are shifted from each other, it makes change in force gradual at the time of peeling off the locking portion 60 along the vertical direction. This enables to make the waist portion 20 difficult to be torn.

### Modified Examples

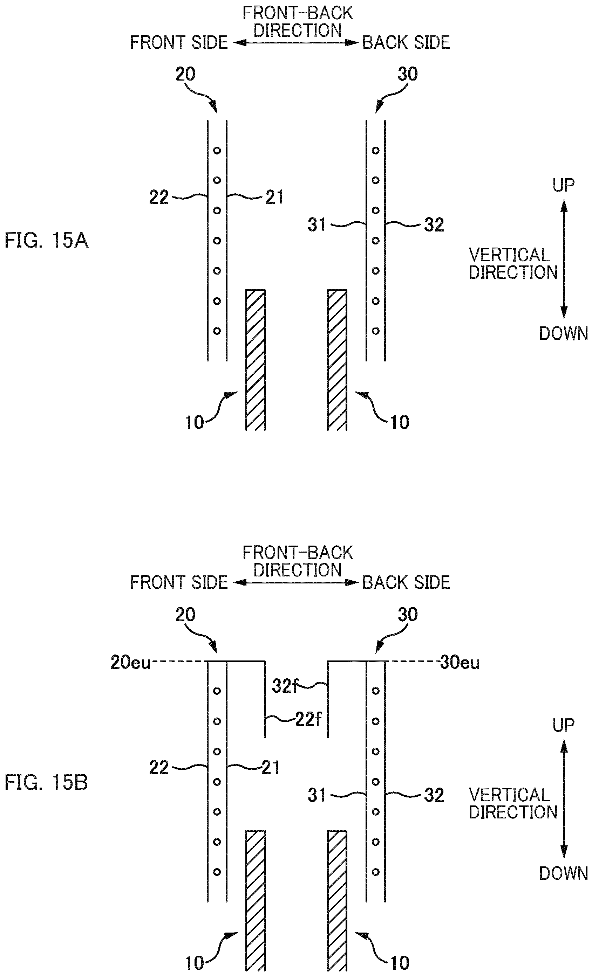

The front waist portion 20 and the back waist portion 30 of the diaper 1 can also be deformed as follows. FIGS. 15A and 15B are schematic cross-sectional views showing modified examples of the waist portion 20 (30).

FIG. 15A shows a case where the skin surface sheet 25 (35) is not provided on the skin-side surface of the waist portion 20 (30). In this case, the inner-layer sheet 21 (31) is a skin-side sheet member arranged the farthest on the skin side of the waist portion 20 (30). In addition, in FIG. 15B, a part of the outer-layer sheet 22 (32) extends upward in the vertical direction, and the extended portion is folded back inward in the front-back direction at an upper end 20eu (30eu) of the waist portion 20 (30), and a folded-back portion 22f (32f) overlaid on the skin side of the inner-layer sheet 21 (31) is formed. In this case, in the region where the folded-back portion 22f (32f) is present, the folded-back portion 22f (32f) is a skin-side sheet member arranged the farthest on the skin side of the waist portion 20 (30).

In both cases, by configuring the diaper 1 so that the sum of the basis weight of the skin-side sheet member and the basis weight of the non-skin-side sheet member is equal to or less than 32 g/m² and so that the tearing strength of the skin-side sheet member in the lateral direction is stronger than the peeling strength of the locking portions 60, it is possible to suppress the tearing of the waist portions 20 and 30 in the operation of removing the diaper 1 from the wearer's body.

### Other Embodiments

Although the above embodiment of the present invention has been described, but the above-described embodiment is intended to facilitate the understanding of the present invention and are not intended to limit the interpretation of the present invention.

In the above-described embodiment, a diaper in which the front waist portion 20 and the back waist portion 30 are separate members has been exemplified. However, the front waist portion 20 and the back waist portion 30 may be a single member that is continuous by a crotch portion provided between the front waist portion 20 and the back waist portion 30.

In the above-described embodiment, the leak-proof wall portions 40 are formed by folding the sheet member 14. That is, the leak-proof wall portions 40 are formed in an integrated manner with the sheet member 14. However, the present invention is not limited thereto. For example, the leak-proof wall portions 40 may be formed of a separate sheet member that is different from the sheet member 14.

### REFERENCE SIGNS LIST

1: diaper (absorbent article),
10: absorbent main body, 11: absorbent core, 11C: narrow portion,
12: skin-side sheet, 13: non-skin-side sheet,
14: sheet member, 15: core-wrapping sheet,
20: front waist portion,
20U: upper end region, 20C: central region, 20D: lower end region,
21: inner-layer sheet,
22: outer-layer sheet, 22f: folded-back portion, 23: waist elastic member,
25: skin surface sheet (skin-side sheet member),
30: back waist portion, 30A: overlapping portion, 30B: extension portion,
30U: upper end region, 30C: central region, 30D: lower end region,
31: inner-layer sheet,
32: outer-layer sheet, 32f: folded-back portion, 33: waist elastic member,
34: extension-portion elastic member, 341: inclined portion, 342: straight portion,
35: skin surface sheet (skin-side sheet member),
40: leak-proof wall portion, 41: leak-proof-wall stretchable member,
60: locking portion,
61: upper end, 61a: upper end, 61b: upper end, 62: lower end,
63: peeling point,
601: first pattern, 602: second pattern,
A60: adjacent region,
BH: waist opening, LH: leg opening

## Claims

1. A underpants-shaped disposable diaper (1) having a vertical direction, a lateral direction, and a front-back direction that intersect each other,
the underpants-shaped disposable diaper (1) comprising:
a liquid-absorbent absorbent main body (10);
a front waist portion (20) and a back waist portion (30) that are provided on a non-skin side of the absorbent main body (10); and
a pair of locking portions (60) that lock two lateral end portions of the front waist portion (20) and two lateral end portions of the back waist portion (30),
in each of the front waist portion (20) and the back waist portion (30),
a sum of a basis weight of a skin-side sheet member (25, 35) arranged the farthest on a skin side and a basis weight of a sheet member (22, 32) arranged the farthest on a non-skin side being 32 g/m² or less,
the skin-side sheet member (25, 35) overlapping the locking portions (60) in two end portions in the lateral direction,
the skin-side sheet member (25, 35) including a non-joining portion in a region (A60) adjacent to inside of the locking portion (60) in the lateral direction,
the non-joining portion being a portion that is not joined to a sheet member overlaid on a non-skin side of the skin-side sheet member (25, 35),
a tearing strength for tearing the skin-side sheet member (25, 35) along the lateral direction being greater than a peeling strength of the locking portion (60).

2. The underpants-shaped disposable diaper (1) according to claim **1,** wherein
in at least one of an upper end region and a lower end region of the locking portion (60),
of at least one of the front waist portion (20) and the back waist portion (30),
a basis weight of a sheet member arranged the farthest on the skin side is equal to or more than a basis weight of a sheet member arranged the farthest on the non-skin side.

3. The underpants-shaped disposable diaper (1) according to claim 2, wherein
in both the upper end region and the lower end region of the locking portion (60),
of at least one of the front waist portion (20) and the back waist portion (30),
a basis weight of a sheet member arranged the farthest on the skin side is equal to or more than a basis weight of a sheet member arranged the farthest on the non-skin side.

4. The underpants-shaped disposable diaper (1) according to any one of claims 1 to 3, wherein
the front waist portion (20) and the back waist portion (30) each have a waist elastic member (23), and
the waist elastic member (23) is arranged on the non-skin side with respect to a central position in a thickness direction of at least one of the front waist portion (20) and the back waist portion (30).

5. The underpants-shaped disposable diaper (1) according to any one of claims 1 to 4, wherein
in at least one of the front waist portion (20) and the back waist portion (30),
a thickness of a sheet member arranged the farthest on the skin side is equal to or more than a thickness of a sheet member arranged the farthest on the non-skin side.

6. The underpants-shaped disposable diaper (1) according to any one of claims 1 to 5, wherein
an average value of a width of the locking portion (60) in the lateral direction is 3 mm or less.

7. The underpants-shaped disposable diaper (1) according to any one of claims 1 to 6,wherein
in at least one of the front waist portion (20) and the back waist portion (30),
a number of overlaid sheet members that are overlaid in a thickness direction in a certain region in the vertical direction
is different from
a number of overlaid sheet members that are overlaid in the thickness direction in another region in the vertical direction.

8. The underpants-shaped disposable diaper (1) according to claim 7, wherein
in at least one of the front waist portion (20) and the back waist portion (30),
the locking portion (60) has a different locking pattern for each of regions in which the number of overlaid sheet members overlaid in the thickness direction is different.

9. The underpants-shaped disposable diaper (1) according to claim 8, wherein
in at least one of the front waist portion (20) and the back waist portion (30),
in the vertical direction, a position where the locking pattern changes is different from a position where the number of overlaid sheet members that are overlaid in the thickness direction changes.

10. The underpants-shaped disposable diaper (1) according to any one of claims 7 to 9, wherein
at least one of the front waist portion (20) and the back waist portion (30) includes:
a first region having a larger number of overlaid sheet members that are overlaid in the thickness direction; and
a second region having a smaller number of overlaid sheet members that re overlaid in the thickness direction, than in the first region, and
a peeling strength of the locking portion (60) in the first region is weaker than a peeling strength of the locking portion (60) in the second region.

11. The underpants-shaped disposable diaper (1) according to any one of claims 1 to 10, wherein
in the region (A60) adjacent to inside of the locking portion (60) in the lateral direction,
the skin-side sheet member (25, 35) and the sheet member overlaid on the non-skin side of the skin-side sheet member (25, 35) include a joining portion where the skin-side sheet member and the sheet member are joined.

## Patentansprüche

1. Unterhosen-förmige Einwegwindel (1), die eine vertikale Richtung, eine laterale Richtung und eine Vorn-Hinten-Richtung aufweist, die einander schneiden,
wobei die Unterhosen-förmige Einwegwindel (1) Folgendes umfasst:
einen flüssigkeitsabsorbierenden absorbierenden Hauptkörper (10);
einen vorderen Taillenteil (20) und einen hinteren Taillenteil (30), die auf einer Nicht-Hautseite des absorbierenden Hauptkörpers (10) bereitgestellt sind; und
ein Paar von Verschlussteilen (60), die zwei laterale Endteile des vorderen Taillenteils (20) und zwei laterale Endteile des hinteren Taillenteils (30) verschließen,
wobei in jedem des vorderen Taillenteils (20) und des hinteren Taillenteils (30),
eine Summe eines Flächengewichts eines Hautseitenlagenelements (25, 35), das am weitesten auf einer Hautseite angeordnet ist, und eines Flächengewichts eines Lagenelements (22, 32), das am weitesten auf einer Nicht-Hautseite angeordnet ist, 32 g/m² oder weniger beträgt,
wobei das Hautseitenlagenelement (25, 35) die Verschlussteile (60) in zwei Endteilen in der lateralen Richtung überlappt,
wobei das Hautseitenlagenelement (25, 35) einen nicht-verbindenden Teil in einem Bereich (A60), angrenzend an die Innenseite des Verschlussteils (60) in der lateralen Richtung, einschließt,
wobei der nicht-verbindende Teil ein Teil ist, der nicht mit einem Lagenelement verbunden ist, das auf einer Nicht-Hautseite des Hautseitenlagenelements (25, 35) überlagert ist,
wobei eine Reißfestigkeit zum Reißen des Hautseitenlagenelements (25, 35) entlang der lateralen Richtung größer ist als eine Abziehfestigkeit des Verschlussteils (60).

2. Unterhosen-förmige Einwegwindel (1) nach Anspruch 1, wobei
in mindestens einem eines oberen Endbereichs und eines unteren Endbereichs des Verschlussteils (60)
von mindestens einem des vorderen Taillenteils (20) und des hinteren Taillenteils (30)
ein Flächengewicht eines Lagenelements, das am weitesten auf der Hautseite angeordnet ist, gleich ist wie oder größer ist als ein Flächengewicht eines Lagenelements, das am weitesten auf der Nicht-Hautseite angeordnet ist.

3. Unterhosen-förmige Einwegwindel (1) nach Anspruch 2, wobei
in sowohl dem oberen Endbereich als auch dem unteren Endbereich des Verschlussteils (60)
von mindestens einem des vorderen Taillenteils (20) und des hinteren Taillenteils (30)
ein Flächengewicht eines Lagenelements, das am weitesten auf der Hautseite angeordnet ist, gleich ist wie oder größer ist als ein Flächengewicht eines Lagenelements, das am weitesten auf der Nicht-Hautseite angeordnet ist.

4. Unterhosen-förmige Einwegwindel (1) nach einem der Ansprüche 1 bis 3, wobei
der vordere Taillenteil (20) und der hintere Taillenteil (30) jeweils ein elastisches Taillenelement (23) aufweisen und
das elastische Taillenelement (23) auf der Nicht-Hautseite in Bezug auf eine mittlere Position in einer Dickenrichtung von mindestens einem des vorderen Taillenteils (20) und des hinteren Taillenteils (30) angeordnet ist.

5. Unterhosen-förmige Einwegwindel (1) nach einem der Ansprüche 1 bis 4, wobei
in mindestens einem des vorderen Taillenteils (20) und des hinteren Taillenteils (30)
eine Dicke eines Lagenelements, das am weitesten auf der Hautseite angeordnet ist, gleich ist wie oder größer ist als eine Dicke eines Lagenelements, das am weitesten auf der Nicht-Hautseite angeordnet ist.

6. Unterhosen-förmige Einwegwindel (1) nach einem der Ansprüche 1 bis 5, wobei
ein durchschnittlicher Wert einer Breite des Verschlussteils (60) in der lateralen Richtung 3 mm oder weniger beträgt.

7. Unterhosen-förmige Einwegwindel (1) nach einem der Ansprüche 1 bis 6, wobei
in mindestens einem des vorderen Taillenteils (20) und des hinteren Taillenteils (30)
sich eine Anzahl von übereinandergelegten Lagenelementen, die in einer Dickenrichtung übereinandergelegt sind, in einem bestimmten Bereich in der vertikalen Richtung
von
einer Anzahl von übereinandergelegten Lagenelementen, die in der Dickenrichtung übereinandergelegt sind, in einem anderen Bereich in der vertikalen Richtung unterscheidet.

8. Unterhosen-förmige Einwegwindel (1) nach Anspruch 7, wobei
in mindestens einem des vorderen Taillenteils (20) und des hinteren Taillenteils (30)
der Verschlussteil (60) ein anderes Verschlussmuster für jeden von Bereichen aufweist, in dem sich die Anzahl von übereinandergelegten Lagenelementen, die in der Dickenrichtung übereinandergelegt sind, unterscheidet.

9. Unterhosen-förmige Einwegwindel (1) nach Anspruch 8, wobei
in mindestens einem des vorderen Taillenteils (20) und des hinteren Taillenteils (30)
in der vertikalen Richtung sich eine Position, wo sich das Verschlussmuster ändert, von einer Position unterscheidet, wo sich die Anzahl von übereinandergelegten Lagenelementen, die in der Dickenrichtung übereinandergelegt sind, ändert.

10. Unterhosen-förmige Einwegwindel (1) nach einem der Ansprüche 7 bis 9, wobei
mindestens einer des vorderen Taillenteils (20) und des hinteren Taillenteils (30) Folgendes einschließt:
einen ersten Bereich, der eine größere Anzahl von übereinandergelegten Lagenelementen, die in der Dickenrichtung übereinandergelegt sind, aufweist; und
einen zweiten Bereich, der eine kleinere Anzahl von übereinandergelegten Lagenelementen, die in der Dickenrichtung übereinandergelegt sind, aufweist als in dem ersten Bereich, und
eine Abziehfestigkeit des Verschlussteils (60) in dem ersten Bereich schwächer ist als eine Abziehfestigkeit des Verschlussteils (60) in dem zweiten Bereich.

11. Unterhosen-förmige Einwegwindel (1) nach einem der Ansprüche 1 bis 10, wobei
in dem Bereich (A60), angrenzend an die Innenseite des Verschlussteils (60) in der lateralen Richtung,
das Hautseitenlagenelement (25, 35) und das Lagenelement, das auf der Nicht-Hautseite des Hautseitenlagenelements (25, 35) überlagert ist, einen verbindenden Teil einschließen, wo das Hautseitenlagenelement und das Lagenelement verbunden sind.

## Revendications

1. Couche jetable en forme de culotte (1) ayant une direction verticale, une direction latérale et une direction allant dans le sens avant-arrière qui se croisent les unes les autres,
la couche jetable en forme de culotte (1) comportant :
un corps principal absorbant qui absorbe les liquides (10) ;
une partie avant au niveau de la taille (20) et une partie arrière au niveau de la taille (30) qui sont mises en œuvre sur un côté non orienté vers la peau du corps principal absorbant (10) ; et
une paire de parties de blocage (60) qui bloquent deux parties d'extrémité latérales de la partie avant au niveau de la taille (20) et deux parties d'extrémité latérales de la partie arrière au niveau de la taille (30),
dans chacune de la partie avant au niveau de la taille (20) et de la partie arrière au niveau de la taille (30),
une somme d'une masse surfacique d'un élément formant feuille côté orienté vers la peau (25, 35) agencé le plus loin sur un côté orienté vers la peau et d'une masse surfacique d'un élément formant feuille (22, 32) agencé le plus loin sur un côté non orienté vers la peau étant de 32 g/m² ou moins,
l'élément formant feuille côté orienté vers la peau (25, 35) chevauchant les parties de blocage (60) dans deux parties d'extrémité dans la direction latérale,
l'élément formant feuille côté orienté vers la peau (25, 35) comprenant une partie de non-assemblage dans une région (A60) adjacente par rapport à l'intérieur de la partie de blocage (60) dans la direction latérale,
la partie de non-assemblage étant une partie qui n'est pas assemblée à un élément formant feuille superposé sur un côté non orienté vers la peau de l'élément formant feuille côté orienté vers la peau (25, 35),
une résistance à la déchirure pour déchirer l'élément formant feuille côté orienté vers la peau (25, 35) le long de la direction latérale est supérieure à une résistance au pelage de la partie de blocage (60).

2. Couche jetable en forme de culotte (1) selon la revendication 1, dans laquelle
dans au moins l'une parmi une région d'extrémité supérieure et une région d'extrémité inférieure de la partie de blocage (60),
d'au moins l'une parmi la partie avant au niveau de la taille (20) et la partie arrière au niveau de la taille (30),
une masse surfacique d'un élément formant feuille agencé le plus loin sur le côté orienté vers la peau est égale ou supérieure à une masse surfacique d'un élément formant feuille agencé le plus loin sur le côté non orienté vers la peau.

3. Couche jetable en forme de culotte (1) selon la revendication 2, dans laquelle
dans à la fois la région d'extrémité supérieure et la région d'extrémité inférieure de la partie de blocage (60),
d'au moins l'une parmi la partie avant au niveau de la taille (20) et la partie arrière au niveau de la taille (30),
une masse surfacique d'un élément formant feuille agencé le plus loin sur le côté orienté vers la peau est égale ou supérieure à une masse surfacique d'un élément formant feuille agencé le plus loin sur le côté non orienté vers la peau.

4. Couche jetable en forme de culotte (1) selon l'une quelconque des revendications 1 à 3, dans laquelle
la partie avant au niveau de la taille (20) et la partie arrière au niveau de la taille (30) ont chacune un élément élastique au niveau de la taille (23), et
l'élément élastique au niveau de la taille (23) est agencé sur le côté non orienté vers la peau par rapport à une position centrale dans une direction allant dans le sens de l'épaisseur d'au moins l'une parmi la partie avant au niveau de la taille (20) et la partie arrière au niveau de la taille (30).

5. Couche jetable en forme de culotte (1) selon l'une quelconque des revendications 1 à 4, dans laquelle
dans au moins l'une parmi la partie avant au niveau de la taille (20) et la partie arrière au niveau de la taille (30),
une épaisseur d'un élément formant feuille agencé le plus loin sur le côté orienté vers la peau est égale ou supérieure à une épaisseur d'un élément formant feuille agencé le plus loin sur le côté non orienté vers la peau.

6. Couche jetable en forme de culotte (1) selon l'une quelconque des revendications 1 à 5, dans laquelle
une valeur moyenne d'une largeur de la partie de blocage (60) dans la direction latérale est de 3 mm ou moins.

7. Couche jetable en forme de culotte (1) selon l'une quelconque des revendications 1 à 6, dans laquelle
dans au moins l'une parmi la partie avant au niveau de la taille (20) et la partie arrière au niveau de la taille (30),
un certain nombre d'éléments formant feuille superposés qui sont superposés dans une direction allant dans le sens de l'épaisseur dans une certaine région dans le sens vertical
est différent par rapport à
un certain nombre d'éléments formant feuille superposés qui sont superposés dans la direction allant dans le sens de l'épaisseur dans une autre région dans le sens vertical.

8. Couche jetable en forme de culotte (1) selon la revendication 7, dans laquelle
dans au moins l'une parmi la partie avant au niveau de la taille (20) et la partie arrière au niveau de la taille (30),
la partie de blocage (60) a une configuration de blocage différente pour chacune des régions dans lesquelles le nombre d'éléments formant feuille superposés qui sont superposés dans le sens de l'épaisseur est différent.

9. Couche jetable en forme de culotte (1) selon la revendication 8, dans laquelle
dans au moins l'une parmi la partie avant au niveau de la taille (20) et la partie arrière au niveau de la taille (30),
dans le sens vertical, une position dans laquelle la configuration de blocage change est différente d'une position dans laquelle le nombre d'éléments formant feuille superposés qui sont superposés dans la direction allant dans le sens de l'épaisseur change.

10. Couche jetable en forme de culotte (1) selon l'une quelconque des revendications 7 à 9, dans laquelle
dans au moins l'une parmi la partie avant au niveau de la taille (20) et la partie arrière au niveau de la taille (30) comprend :
une première région ayant un plus grand nombre d'éléments formant feuille superposés qui sont superposés dans la direction allant dans le sens de l'épaisseur ; et
une deuxième région ayant un plus petit nombre d'éléments formant feuille superposés qui sont superposés dans la direction allant dans le sens de l'épaisseur, que dans la première région, et
une résistance au pelage de la partie de blocage (60) dans la première région est plus faible qu'une résistance au pelage de la partie de blocage (60) dans la deuxième région.

11. Couche jetable en forme de culotte (1) selon l'une quelconque des revendications 1 à 10, dans laquelle
dans la région (A60) adjacente par rapport à l'intérieur de la partie de blocage (60) dans la direction latérale,
l'élément formant feuille côté orienté vers la peau (25, 35) et l'élément formant feuille superposé sur le côté non orienté vers la peau de l'élément formant feuille côté orienté vers la peau (25, 35) comprennent une partie **d'assemblage** où l'élément formant feuille côté orienté vers la peau et l'élément formant feuille sont assemblés.
